# EUROPÄISCHE PATENTSCHRIFT

(11) **EP 1 771 242 B1**
(45) Veröffentlichungstag und Bekanntmachung des Hinweises auf die Patenterteilung: **22.04.2020**
(21) Anmeldenummer: 05761702.9
(22) Anmeldetag: 15.07.2005
(51) Int. Cl.: C12N 15/10

(54) **VERFAHREN ZUR EFFIZIENTEN ISOLIERUNG VON NUKLEINSÄUREN**
METHOD FOR EFFICIENT ISOLATION OF NUCLEIC ACIDS
PROCEDE PERMETTANT D'ISOLER EFFICACEMENT DES ACIDES NUCLEIQUES

(30) Priorität: 15.07.2004 DE 102004034474
(43) Veröffentlichungstag der Anmeldung: 11.04.2007
(73) Patentinhaber: QIAGEN GmbH, 40724 Hilden (DE)
(72) Erfinder: SINGER, Thorsten, 42697 Solingen (DE)
(74) Vertreter: Roth, Carla
(86) Internationale Anmeldenummer: PCT/EP2005/007726
(87) Internationale Veröffentlichungsnummer: WO 2006/008085

(56) Entgegenhaltungen:
- EP-A- 1 321 176
- DE-A1- 19 856 064
- US-A- 4 647 380
- US-B1- 6 277 648
- US-B1- 6 277 648

## Beschreibung

Die vorliegende Offenbarung betrifft eine Vorrichtung und ein Verfahren unter Verwendung der Vorrichtung zur effizienteren Aufreinigung von Nukleinsäuren sowie ein Kit zur Durchführung dieses Verfahrens.

Wie aus dem Stand der Technik bekannt ist, erfolgt die Isolierung von Nukleinsäuren aus komplexen biologischen Ausgangsmaterialien unter stark denaturierenden und reduzierenden Bedingungen. Die Nukleinsäuren enthaltenden Ausgangsmaterialien werden teilweise unter Verwendung von proteinabbauenden Enzymen aufgeschlossen, und die austretenden Nukleinsäurefraktionen durch einen Phenol/Chloroform-Extraktionsschritt gereinigt. Die Nukleinsäuren können anschließend mittels Dialyse oder Ethanolpräzipitation aus der wässrigen Phase gewonnen werden (J. Sambrock, E.F. Fritsch und T. Maniatis, 1989, Cold Spring Harbor, "Molecular Cloning").

Die für diese Isolierung von Nukleinsäuren aus dem Stand der Technik bekannten Verfahren weisen jedoch den Nachteil auf, dass sie zeitaufwendig sind und einen erheblichen apparativen Aufwand erfordern. Außerdem können sich solche Methoden auf Grund der verwendeten Chemikalien wie Phenol und Chloroform als gesundheitsgefährdend erweisen.

Um die gesundheitsschädigende und aufwendige Phenol-/Chloroform-Extraktion von Nukleinsäuren zu umgehen, verbunden mit einer zusätzlichen Reduzierung der zeitlichen experimentellen Aufwendungen, wurden in der Vergangenheit verschiedene alternative Verfahren zur Isolierung von Nukleinsäuren aus unterschiedlichen biologischen Ausgangsmaterialien entwickelt.

Hierzu gehören Verfahren, die erstmals auf einem von Vogelstein und Gillespie (Proc. Natl. Acad. Sci. USA, 1979, 76, 615-619) beschriebenen Verfahren zur präparativen und analytischen Reinigung von DNA-Fragmenten aus Agarosegelen basieren. Diese Methode kombiniert die Auflösung der die zu isolierende DNA-Bande enthaltende Agarose in einer gesättigten Lösung eines chaotropen Salzes (NaJ) mit einer anschließenden DNA-Bindung an Glaspartikel.

Die an den Glaspartikeln fixierte DNA wird anschließend mit einer Waschlösung (20 mM Tris HCl (pH 7,2); 200 mM NaCl, 2 mM EDTA; 50% v/v Ethanol) gewaschen und dann von den Trägerpartikeln eluiert. Diese Methode wurde im Laufe der Zeit vielfach modifiziert und wird zum gegenwärtigen Zeitpunkt für unterschiedliche Verfahren der Extraktion und Reinigung von Nukleinsäuren unterschiedlichsten Ursprungs benutzt.

Aktuell existiert eine Vielzahl von Reagenziensystemen (Kits), vor allem zur Reinigung von DNA-Fragmenten aus Agarosegelen und auch für die Isolierung von Plasmid-DNA aus bakteriellen Lysaten, die mit einer zusätzlichen Isolierung von längerkettigen Nukleinsäuren (genomische DNA, zelluläre RNA) aus Blut, Geweben oder auch Zellkulturen verbunden ist.

Diese kommerziell verfügbaren Kits basieren auf dem hinlänglich bekannten Prinzip der Bindung von Nukleinsäuren an mineralische Träger unter Anwesenheit von Lösungen unterschiedlicher chaotroper Salze, wobei die Trägermaterialien zum Beispiel Suspensionen aus fein gemahlenem Glaspulver (z. B. Glasmilk, BIO 101, La Jolla, CA), Diatomeenerden (Fa. Sigma) oder auch Silicagelen (Qiagen, DE 41 39 664 A1) enthalten.

Zusätzliche verschiedenartige Verfahren zur Isolierung von Nukleinsäuren wurden im Stand der Technik genutzt, um die Ausgangsmaterialien mittels eines chaotropen Puffers an eine DNA-bindende feste Phase zu binden (US 5,234,809). Die chaotropen Puffer werden dabei sowohl für die Lyse des Ausgangsmaterials als auch für die Bindung der Nukleinsäuren an die feste Phase benutzt.

Mit diesem Verfahren können Nukleinsäuren aus kleinen Probenmengen, speziell bei Benutzung zur Isolierung viraler Nukleinsäuren verwendet werden. Die Inkubation des Ausgangsmaterials mit einem chaotropen Puffer und der DNA-bindenden festen Phase beinhaltet den Nachteil, dass der durch die chaotropen Puffer zu realisierende Zellaufschluss nicht für alle Materialien einsetzbar ist und insbesondere auch für größere Mengen an Ausgangsmaterialien nur extrem ineffizient und unter einem großen Zeitaufwand eingesetzt werden kann. Daher werden oft zusätzlich mechanische Homogenisierungsverfahren eingesetzt (z. B. bei der Isolierung der DNA aus Gewebeproben). Verschieden hohe Konzentrationen unterschiedlicher chaotroper Puffer müssen dabei für verschiedene Fragestellungen eingesetzt werden und können somit - naturgemäß - nicht universell zur Verwendung kommen.

Um die ggf. schwierige Lyse des Ausgangsmaterials zu vereinfachen, kann für die Nukleinsäureisolierung eine Reihe von kommerziell verfügbaren Produkten benutzt werden, die aber dann nicht mehr auf einem einfach handhabbaren sog. "Single Tube" - Verfahren basieren.

Die für die nachfolgende Bindung der Nukleinsäuren (z. B. an Zentrifugationsmembranen) notwendigen chaotropen Salze müssen nach erfolgter Lyse dem Lyseansatz innerhalb eines besonderen Verfahrensschritts zugegeben werden. Diese chaotropen Salze können auf der anderen Seite aber nicht Bestandteil des Lysepuffers sein, da die proteinzerstörende Funktion den chaotropen Substanzen inhärent innewohnt und diese die für eine effiziente Lyse notwendigen proteolytischen Enzyme ebenfalls zerstören würden.

Die oben beschriebenen und aus dem Stand der Technik bekannten Methoden der Nukleinsäureisolierung unter Verwendung chaotroper Salze haben sich freilich weltweit durchgesetzt und werden mittels kommerziell verfügbarer Produkte millionenfach eingesetzt.

Nach dem Prinzip der Lyse des Ausgangsmaterials werden in einer einfachen Durchführung die Nukleinsäuren an die feste Phase einer Glas- oder Silicamembran gebunden, die sich in einem Zentrifugationssäulchen an einer Trägersubstanz befindet. Anschließend werden die gebundenen Nukleinsäuren mit einem Puffer geringerer lonenstärke eluiert.

In der Fachwelt wurde das physico-chemische Prinzip der Bindung von Nukleinsäuren an mineralische Träger in Anwesenheit chaotroper Salze aufgeklärt. Es wurde postuliert, dass das Binden der Nukleinsäuren an die Oberflächen der mineralischen Träger auf einer Zerstörung der übergeordneten Strukturen des wässrigen Milieus beruht, durch welche die Nukleinsäuren an die Oberfläche von mineralischen Materialien, insbesondere von Glas- bzw. Silicapartikeln, adsorbieren.

Bei hohen Konzentrationen der chaotropen Salze verläuft die Reaktion fast quantitativ. Aus diesem Grund ist eine Pufferkomposition mit hohen lonenstärken chaotroper Salze für die Bindung von Nukleinsäuren an eine nukleinsäurenbindende feste Phase von Bedeutung.

Für die Bindung der Nukleinsäuren an die jeweiligen Trägeroberflächen enthält die Pufferlösung als Hauptkomponente mindestens ein chaotropes Salz. Dieses beinhaltet unter Umständen schon den Lysepuffer oder bei Systemen unter Einbeziehung proteolytischer Enzyme einen notwendigen Bindungspuffer, welcher nach der erfolgten Lyse des Ausgangsmaterials zugegeben wird.

Die Basis für chaotrope Salze sind die Reihen von Hofmeister zur Aussalzung von negativ geladenen, neutralen oder basischen Eiweißlösungen. Die chaotropen Salze sind dadurch charakterisiert, dass sie Proteine denaturieren, die Löslichkeit unpolarer Substanzen in Wasser erhöhen sowie hydrophobe Wechselwirkungen zerstören. Diese Eigenschaften bewirken auch mit Puffersystemen chaotroper Salze die Zerstörung übergeordneter Strukturen des wässrigen Milieus, um so die Bindung der Nukleinsäuren an ausgewählte feste Phasen zu vermitteln.

Zu den bekanntesten Vertretern chaotroper Salze zur Nukleinsäureisolierung gehören Natriumperchlorat, Natriumjodid, Kaliumjodid, Guanidin-Isothiocyanat und Guanidin-Hydrochlorid. Sie sind jedoch kostenintensiv und zudem teilweise toxisch oder ätzend.

Aus dem Stand der Technik ist ein Verfahren zur Isolierung von DNA aus Geweben und Zelllinien beschrieben, bei dem die Zellen in einem Guanidin-Hydrochlorid enthaltenden Puffer dispergiert und in Ethanol gefällt werden (Analytical Biochemistry 162, 1987, 463). Dieses Verfahren ist auf der einen Seite für Kontaminationen anfällig, auf der anderen Seite kann aber ein brauchbares Nukleinsäure-Produkt innerhalb weniger Stunden isoliert werden.

Zusätzlich ist aus dem Stand der Technik ein Verfahren zur Isolierung von Nukleinsäuren unter Verwendung antichaotroper Substanzen bekannt. So kann durch die Zugabe antichaotroper Salze in ein Lyse/Bindungspuffersystem ebenfalls eine verbesserte Isolierung von Nukleinsäuren erreicht werden. Zu den antichaotropen Komponenten gehören Ammonium-, Cäsium-, Natrium- und/oder Kaliumsalze, vorzugsweise Ammoniumchlorid. Bei der Verwendung der Lyse/Bindungspuffersysteme, ohne den Bestandteil von chaotropen Salzen, können Nukleinsäuren, insbesondere genomische DNA, an ein mineralisches Trägermaterial gebunden werden und unter den üblichen Reaktionsbedingungen auch eluiert werden.

Es wurde weiterhin gefunden, dass mit Lyse/Bindungspuffern, deren Hauptkomponenten z. B. Ammoniumsalze anstelle chaotroper Salze sind, bei Extraktionen genomischer DNA aus unterschiedlich komplexen Ausgangsmaterialien (z. B. Blut, Gewebe, Pflanzen) unter Benutzung der bisher üblichen (anderen) Reaktionskomponenten und Trägermaterialien sowie bei gleichem Reaktionsverlauf, mindestens dieselben quantitativen sowie qualitativen Resultate erreicht werden.

So konnte beobachtet werden, dass mit einem Salz, welches Proteine nicht denaturiert, sondern stabilisiert sowie hydrophobe Wechselwirkungen nicht zerstört, sondern verstärkt, es genauso möglich ist, Nukleinsäuren auch aus komplexen Ausgangsmaterialien zu isolieren.

Für die Bindung der Nukleinsäuren an die festen Träger reichen geringe Konzentrationen von Salzen (kleiner 1 M) aus. Bei bestimmten Applikationen werden Konzentrationen kleiner 0,5 M bevorzugt, wobei für die quantitative Isolierung von Nukleinsäuren aus größeren Mengen an Ausgangsmaterialien höhere lonenkonzentrationen erforderlich sind.

Diese aus dem Stand der Technik bekannten Lyse/Bindungspuffersysteme, die mindestens eine antichaotrope Salzkomponente aufweisen, sind somit in der Lage, Nukleinsäuren an feste Phasen zu binden, die eine negativ geladene Oberfläche besitzen, oder Oberflächen enthalten, die in der Lage sind ein negatives Ladungspotential aufzuweisen.

Der Reaktionsablauf der Nukleinsäureisolierung aus einem komplexen Ausgangsmaterial wird durch die Durchführung der Lyse des Ausgangsmaterials, dem Binden der Nukleinsäuren, dem Waschen der gebundenen Nukleinsäuren und der Elution der Nukleinsäuren in einem Reaktionsgefäß realisiert, und erfordert mindestens einen Zentrifugationsschritt.

Bei den bisher aus dem Stand der Technik bekannten Verfahren zur Isolierung von Plasmid-DNA mit den üblichen Midi- und Maxi-Systemen handelt es sich allerdings nur um vergrößerte Mini-Präparationen, deren Säulen entweder auf größere Durchmesser vergrößert oder um sehr viele Membranlagen ergänzt wurden, um so die erforderliche Kapazität zu erreichen. Derartige Systeme werden z. Zt. beispielsweise von Herstellern wie Stratagene oder Sigma angeboten.

Die hierbei eingesetzten Glasfasermembranen arbeiten fast ausschließlich unter Verwendung chaotroper Salze zur selektiven Bindung an die Oberfläche der Membran.

Als Hauptnachteil gilt die aufwendige Handhabung (viele Zentrifugationsschritte mit großen Bodenzentrifugen). Dazu kommt ein relativ großes Elutionsvolumen verbunden mit einem großen Totvolumen aufgrund der großen inneren Oberflächen und der daraus resultierenden größeren Volumina der Membranen.

Durch diese für die Erhöhung der Kapazität benötigte Oberfläche ist das Aufarbeiten größerer Ansätze mit einer Vorrichtung basierend auf dem Mini-Format unter Beibehaltung einer chaotropen Bindechemie bislang nicht möglich gewesen.

Auf der anderen Seite wird eine Plasmidisolierung auf der Basis einer alkoholischen Bindechemie bisher lediglich durch die Invisorb® Plasmid Kits der Firma Invitek (Berlin) ermöglicht. Aber auch in diesen Kits werden Säulen verwendet, die aufgrund ihrer Abmessungen nicht für Ansätze im Midi/Maxi-Maßstab geeignet sind. Für diese gelten somit ebenfalls die bereits beschriebenen Nachteile.

EP 1 321 176 A1 beschreibt die Produktion von Plasmid-DNA im Produktionsmaßstab und lehrt, hierzu eine spezielle, monolithische feste Phase einzusetzen.

US 6,277,648 B1 betrifft Vorrichtungen und Verfahren zur Isolierung von Nukleinsäuren, bspw. Plasmid-DNA, aus natürlichen Proben wie insbesondere Zellen, wobei zunächst durch Filtration aus einem Verdau verdaute Probenbestandteile wie Zelltrümmer von den übrigen, flüssigen Bestandteilen des Verdaus abgetrennt werden.

DE 198 56 064 A1 stellt ein Verfahren zur Aufreinigung von DNA, bspw. Plasmid-DNA, aus beliebigen Ausgangsmaterialien bereit, bei dem die Anwendung chaotroper Substanzen und damit einhergehenden Nachteile vermieden werden.

Die **Aufgabe** der vorliegenden Erfindung besteht nunmehr darin, ein Verfahren zur Verfügung zu stellen, das es erlaubt, DNA-Plasmidisolierungen in einem großen Maßstab (in sog. Maxi- bzw. Midi-Präparationen) durchzuführen und dabei den Einsatz zeitaufwendiger Zentrifugationsschritte unter Einsatz großer Bodenzentrifugen zu vermeiden.

Die beanspruchte Erfindung wird durch die zugehörigen Ansprüche bestimmt.

Diese Aufgabe wird erfindungsgemäß durch den Einsatz einer sog. Mini-Spin-Säule, die an sich aus dem Stand der Technik bekannt ist, **gelöst**, die jedoch eine spezielle Membran aufweist, wie sie in den Ansprüchen definiert ist. Erfindungsgemäß ermöglicht die Membran auf der einen Seite einen raschen Durchfluss des Lysat-/Alkoholgemisches und auf der anderen Seite wird eine ausreichend große Binde-/Filterkapazität gewährleistet. Optional kann - je nach Erfordernissen - eine zusätzliche Filterschicht über die Membran gelegt werden, um ein vorzeitiges Verstopfen zu vermeiden.

Gemäß der vorliegenden Erfindung hat die Verwendung der oben beschriebenen Membranen für das erfindungsgemäße Verfahren einen entscheidenden Einfluss auf die DNA-Bindung an die Silicamembran, die sich vorzugsweise in einem Hohlkörper einer zur Chromatographie, insbesondere zur lonenaustauscherchromatographie, geeigneten Säule befinden kann. Hierbei ist zu beachten, dass die Membran über eine geeignete Porenweite verfügt, die einerseits die DNA zurückhalten kann und andererseits einen guten Durchfluss auch bei der Filtration größerer Mengen ohne Verstopfung ermöglicht. Ferner sollte die Innenfläche der Chromatographiesäule mit einem zum lonenaustausch befähigten chromatographischen Material beschichtet sein.

GF/A-, GF/B- und GF/D-Membranen von Whatman sind bekannt. Hierbei handelt es sich um Membranen, die aus Mikro-Glasfasern aufgebaut werden und die in der Hauptsache zu Filtrationszwecken (beispielsweise zur Reinigung von Wasser) eingesetzt werden. Dabei ergibt sich eine abnehmende Durchflussgeschwindigkeit in der Reihe GF/D (2,7 µm) >> GF/A (1.6 µm) > GF/B (1 µm),

Die Bindung der Nukleinsäuren an die Oberfläche mineralischer Träger in Anwesenheit chaotroper Salze erfordert Porenweiten der Membran von ca. 1 µm, um akzeptable Ausbeuten zu erzielen, was die Durchflusszeiten bei großen Volumina so verlängert, dass sich der Bindeschritt recht zeitaufwendig gestalten würde.

Überraschenderweise wurde gefunden, dass Membranen, die Porengrößen in einem Intervall von 2 - 4 µm besitzen, DNA in hoher Ausbeute bei großer Durchlaufgeschwindigkeit binden, wenn eine alternative (nicht chaotrope) Bindechemie eingesetzt wird.

Damit kann der Nachteil der langen Durchflusszeit ausgeschaltet werden. Daher wird für die Bindung der Nukleinsäuren erfindungsgemäß ein aliphatischer Alkohol mit 1 bis 5 Kohlenstoffatomen - vorzugsweise Ethanol oder *iso*-Propanol - eingesetzt, der die Verwendung größerer Membranporen erlaubt und dadurch die Durchfluss- und damit verbunden die Präparationszeit entscheidend verkürzt.

Durch die Ausführung der vorliegenden Erfindung wird es zudem ermöglicht, die Nukleinsäuren mit einer Säule im Miniformat mit einer geringen Menge Puffer zu eluieren.

Das erhaltene Elutionsvolumen unter Verwendung der im Vergleich zu den in den oben genannten Midi- und Maxi-Format eingesetzten relativ kleinen Membranen war dabei mit der eingesetzten Puffermenge identisch. Der Vorteil des konstanten Elutionsvolumens dieser sog. "Schnellpräparationsmethode" besteht darin, dass zur Bestimmung der Gesamtausbeute das tatsächliche Volumen nicht extra bestimmt werden muss. Außerdem ist die DNA-Lösung hoch konzentriert und kann gegebenenfalls einfach verdünnt werden. Im Gegensatz dazu müsste eine sehr verdünnte Lösung, die nach den Verfahren des Standes der Technik gewonnen wird, mit deutlich mehr Aufwand konzentriert werden.

Zur Aufnahme der größeren Volumina für den Midi/Maxi-Maßstab wird die Spinsäule mit einem entsprechenden Extender-Tube versehen. Die offenbarte Vorrichtung zur Aufreinigung von Nukleinsäuren umfasst somit eine Säulenkombination bestehend aus:
a. einem offenen Hohlkörper (1) mit einer Einlassöffnung und einer Auslassöffnung, der auf einem weiteren
b. offenen Hohlkörper (2) mit einer Einlassöffnung und einer Auslassöffnung, mit einer oder mehreren darin angebrachten Membran(en),
angeordnet ist.

Die Säulen-/Extender-Kombination wird dann auf einer Vakuumkammer platziert (z. B. der QIAvac® 6S der Fa. QIAGEN, 40724 Hilden) und die Probe mittels Vakuum durch die Membran gezogen. Anschließend wird die Extender-Tube verworfen und die Mini-Spin-Säule entsprechend bekannter Mini-Präparationsverfahren (z. B. nach QIAprep®, Fa. QIAGEN, 40724 Hilden) weiter bearbeitet.

Der große Vorteil des erfindungsgemäß vorgeschlagenen Verfahrens sowie der dabei eingesetzten Vorrichtung besteht in der kurzen Bearbeitungszeit für die Aufreinigung von Nukleinsäuren sowie in dem Verzicht auf unpraktische, aufwendige Zentrifugationsschritte. Hinzu kommen geringe Elutions- und Totvolumina und die damit verbundenen hohen DNA-Endkonzentrationen.

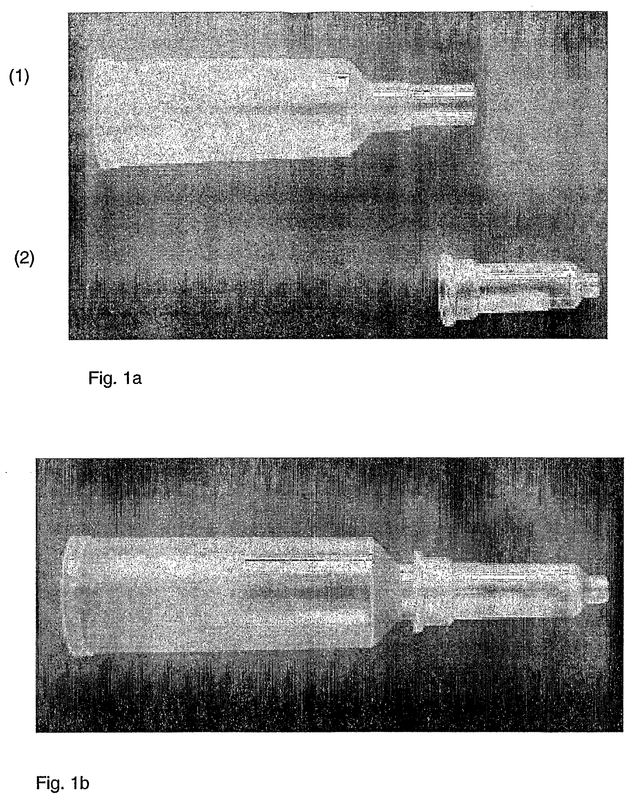

Weitere Einzelheiten der vorliegenden Erfindung werden anhand von beigefügten Zeichnungen (Figuren 1 bis 10) sowie den nachstehend aufgeführten Ausführungsbeispielen näher erläutert.

Es zeigt:
- Fig. 1a: und Fig.1b: eine Säulenkombination aus einem offenen Hohlkörper (1) mit einer Einlassöffnung und einer Auslassöffnung sowie einen weiteren Hohlkörper (2) mit einer Einlassöffnung und einer Auslassöffnung, mit einer enthaltenden Filterschicht im Lumen.
- Fig. 2:: Präparationszeiten (in min) von 4 unterschiedlichen Säulen-Präparationen - mit Säulenmaterial der Firmen Invitek (Produkt 1), SIGMA (Produkt 2) und Stratagene (Produkt 3) sowie der erfindungsgemäß eingesetzten Vorrichtung (Produkt 4) - gemäß Beispiel 1 während der Aufreinigung von Nukleinsäuren.
- Fig. 3:: DNA-Ausbeuten (in µg) von 4 unterschiedlichen Säulen-Präparationen - mit Säulenmaterial der Firmen Invitek (Produkt 1), SIGMA (Produkt 2) und Stratagene (Produkt 3) sowie der erfindungsgemäß eingesetzten Vorrichtung (Produkt 4) - gemäß Beispiel 1 während der Aufreinigung von Nukleinsäuren.
- Fig. 4:: ein Agarosegel mit den Banden von 4 Säulen-Präparationen - mit Säulenmaterial der Firmen Invitek (Produkt 1), SIGMA (Produkt 2) und Stratagene (Produkt 3) sowie der erfindungsgemäß eingesetzten Vorrichtung (Produkt 4).
- Fig. 5:: die Elutionsvolumina (in µl) von 4 Säulen-Präparationen - mit Säulenmaterial der Firmen Invitek (Produkt 1), SIGMA (Produkt 2) und Stratagene (Produkt 3) und der erfindungsgemäß eingesetzten Vorrichtung während des Wechsels des Säulen-Formats von der Midi-Präparation auf die Mini-Präparation
- Fig. 6:: die Präparationszeiten (in min) von 2 unterschiedlichen Säulen-Präparationen - mit dem Säulenmaterial der Firma Invitek und der erfindungsgemäß eingesetzten Vorrichtung - während der Aufreinigung von Nukleinsäuren vor dem Wechsel der Maxi-Präparation auf die Mini-Präparation.
- Fig. 7:: DNA-Ausbeuten (in µg) von 2 unterschiedlichen Säulen-Präparationen - mit Säulenmaterial der Firma Invitek und der erfindungsgemäß eingesetzten Vorrichtung - zur Aufreinigung von Nukleinsäuren während der Reduzierung des Säulen-Formats von der Maxi-Präparation auf die Mini-Präparation.
- Fig. 8:: ein Agarosegel mit den Banden von 2 Säulen-Präparationen - mit Säulenmaterial der Firma Invitek (1) und der erfindungsgemäß eingesetzten Vorrichtung (2).
- Fig. 9:: die Elutionsvolumina (in µl) von 2 Säulen-Präparationen - mit Säulenmaterial der Firma Invitek und der erfindungsgemäß eingesetzten Vorrichtung - während des Wechsels des Säulen-Formats von der Maxi-Präparation auf die Mini-Präparation.
- Fig. 10:: den Einfluss der GF/D Membran mit einer Porengröße von 2,7 µm auf die DNA-Ausbeuten (in µg).

### Beispiel 1:

### Protokoll zur Isolierung von Plasmid-DNA aus E. coli im sog. Midi Maßstab

Protokoll zur Isolierung von Plasmid-DNA aus *E. coli:*
25 ml *E. coli* DH5a - Kultur mit dem Plasmid pCMVβ (DH5α/pCMVβ, Fa. BD Biosciences).
(1) Bakterienpellet in 2 ml Resuspensionspuffer resuspendieren (3min)
(2) Zugabe von 2 ml Lysepuffer und vorsichtig mischen. Ca. 3 min lysieren
(3) Zugabe von 2 ml Neutralisationspuffer, mischen durch Invertieren (1 min)
(4) Rohlysat über eine Silica enthaltende -Filtersäule (QIAfilter QIAGEN), 3 min bei Raumtemperatur inkubieren und das Rohlysat durch das Säulenmaterial pressen.
(5) 10 ml Extension-Tube auf die Spinsäule setzen und auf einer QIAvac® (QIAGEN) positionieren.
(6) Zugabe von 2,5 ml Isopropanol zum Lysat, gut durchmischen und auf die Säule geben.
(7) Mit Hilfe von Vakuum für 2 min das Gemisch durch das Säulenmaterial saugen.
(8) Extension-Tube verwerfen
(9) Die Spin-Säule mit darin enthaltenen Silicamembranen auf eine Collection-Tube (Auffang-Gefäß) setzen.
(10) Waschen durch Zugabe von 750 µl Puffer PE (QIAGEN, handelsüblicher Alkohol-Waschpuffer) und 1 min bei 14 000 Upm zentrifugieren.
(11) Zum Entfernen von Pufferresten noch einmal 1 min bei 14 000 Upm zentrifugieren.
(12) Die Spin-Säule auf eine 1,5 ml Eppendorf-Tube wechseln.
(13) Zur Elution 200 µl eines Elutionspuffers (EB), z. B. dem handelsüblichen Elutionspuffer TE, auf die Membran pipettieren, 1 min stehen lassen und zentrifugieren (1 min bei 14 000 Upm).

Dabei führen die im Protokoll zur Isolierung von Plasmid-DNA aufgeführten Punkte (4), (7) und (10) - (13) zu einer bedeutend schnelleren Isolierung von Plasmid-DNA im Vergleich zu den aus dem Stand der Technik bekannten Systemen. Hinsichtlich des oben angeführten Beispiels wurden Referenz-Präparationen mit folgenden Produkten auf Silica-Basis durchgeführt:
1) Invitek Invisorb Plasmid Midi Kit (Fa. Invitek),
2) SIGMA GeneElute EF Midi Kit (Fa. SIGMA-ALDRICH),
3) Stratagene Strataprep EF Midi (Fa. Stratagene),
4) erfindungsgemäß eingesetzten Vorrichtung
In den Präparationen (2) und (3) wurde, zur besseren Vergleichbarkeit mit den beiden anderen Systemen, die Protokollvariante ohne den im Protokoll angegebenen optionalen Endotoxin-Entfernungsschritt durchgeführt.

Dabei basieren das Invitek Invisorb Plasmid Midi Kit (1) und das mit der erfindungsgemäß eingesetzten Vorrichtung verwendete Verfahren (4) auf einer nicht-chaotropen Silica-Chemie. Die SIGMA (2) und Stratagene (3) Kits hingegen basieren auf einer herkömmlichen Bindung mittels chaotroper Salze.

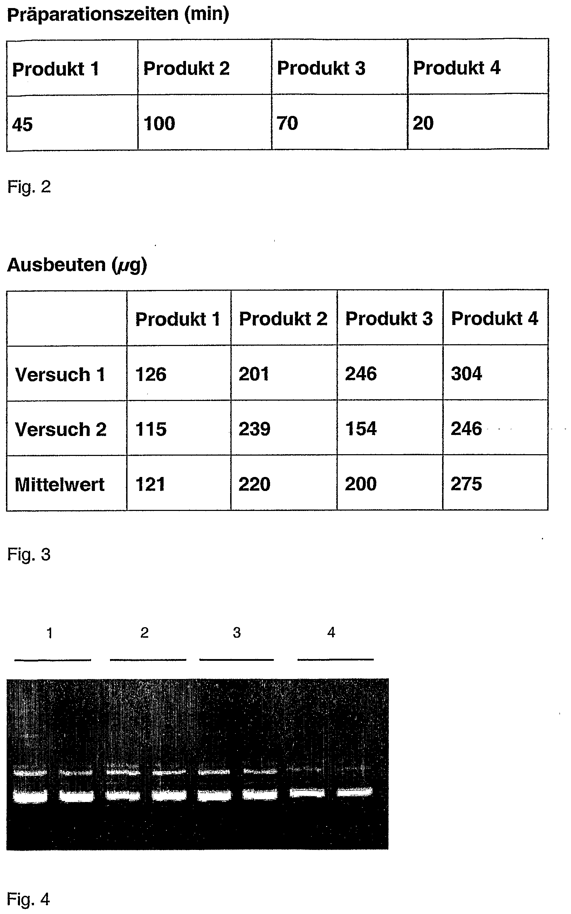

Die Figur 2 zeigt die Präparationszeiten in Minuten (min) von 4 verschiedenen Säulen-Präparationen. Hierbei ermöglicht die erfindungsgemäß eingesetzte Vorrichtung mit 20 min eine deutlich kürzere Präparationszeit zur Isolierung von Nukleinsäuren als die aus dem Stand der Technik bekannten Verfahren von Invitek, SIGMA und Stratagene.

Zusätzlich werden in Figur 3 die DNA-Ausbeuten in Mikrogramm (µg) der 4 Säulen-Präparationen gezeigt, wobei die Ausbeute von 275 µg der erfindungsgemäß eingesetzten Vorrichtung bzw. des mit dieser Vorrichtung durchgeführten Verfahrens (der "Schnellpräparation Midi") deutlich höher als die der Invitek-, SIGMA- oder Stratagene-Präparationen ist.

Die Figur 4 belegt dabei zweifelsfrei, dass die erfindungsgemäß durchgeführten Präparationen von identischer Qualität sind, wie die mit den aus dem Stand der Technik bekannten Verfahren isolierten Proben.

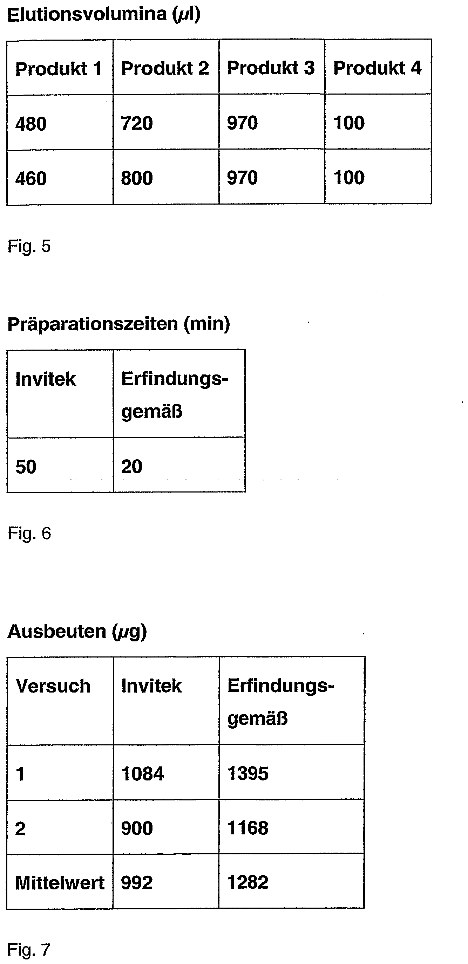

In Figur 5 werden die Elutionsvolumina in Mikroliter (µl) der 4 Säulen-Präparation gegenüber gestellt. Diese verdeutlichen, dass die erfindungsgemäß verwendeten Säulen im Mini-Format mit einer geringeren Menge Puffer eluiert werden können. Durch die kleine Membran gibt es kein Totvolumen, so dass das erhaltene Elutionsvolumen mit dem eingesetzten Volumen an Pufferlösung identisch ist.

Das sehr geringe und konstante Elutionsvolumen der "Schnellpräparations-Methode" stellt für die Bestimmung der Gesamtausbeute sowie für die Folgeanwendungen große Vorteile dar, da die DNA-Lösung hoch konzentriert ist und ggf. einfach verdünnt werden kann. Im Gegensatz dazu müsste eine verdünnte Lösung mit deutlich mehr Aufwand konzentriert werden.

### Beispiel 2:

### Isolierung von Plasmid-DNA aus E. coli im sog. Maxi Maßstab:

Protokoll zur Isolierung von Plasmid-DNA aus E. *coli*
100 ml *E. coli* DH5a - Kultur mit dem Plasmid pCMVβ (DH5α/pCMVβ, Fa. BD Biosciences)
(1) Bakterienpellet in 5 ml Resuspensionspuffer resuspendieren (3min).
(2) Zugabe von 5 ml Lysepuffer und vorsichtig mischen. Ca. 3 min lysieren.
(3) Zugabe von 5 ml Neutralisationspuffer, mischen durch Invertieren (1 min).
(4) Rohlysat über eine Silica enthaltende - Filtersäule (QIAfilter, QIAGEN) geben, 3 min bei Raumtemperatur inkubieren und das Rohlysat durch das Säulenmaterial pressen.
(5) 20 ml Extension-Tube auf die Spinsäule setzen und auf einer QIAvac positionieren.
(6) Zugabe von 7 ml Isopropanol zum Lysat, gut durchmischen und auf die Säule geben.
(7) Mit Hilfe von Vakuum für 2 min das Gemisch durch das Säulenmaterial saugen.
(8) Extension-Tube verwerfen.
(9) Die Spin-Säule mit darin enthaltenen Silicamembranen auf eine Collection-Tube (Auffang-Gefäß) setzen.
(10) Waschen durch Zugabe von 750 µl PE eines handelsüblichen Waschpuffers und 1 min bei 14 000 Upm zentrifugieren.
(11) Zum Entfernen der Pufferreste noch einmal 1 min bei 14 000 Upm zentrifugieren.
(12) Die Spin-Säule auf eine 1,5 ml Eppendorf-Tube wechseln.
(13) Zur Elution 200 µl eines Elutionspuffers (EB), z. B. dem handelsüblichen Elutionspuffer TE, auf die Membran pipettieren, 1 min stehen lassen und zentrifugieren (1 min bei 14 000 Upm).

Bei dem vorstehend ausgeführten Beispiel wurden Referenz-Präparationen mit folgenden Produkten auf Silica-Basis durchgeführt:
1) Invitek Invisorb Plasmid Maxi Kit (Fa. Invitek)
2) erfindungsgemäß eingesetzte Vorrichtung

Die erfindungsgemäß eingesetzte Vorrichtung und das Invitek Produkt beruhen beide auf einer nicht-chaotropen Silica-Chemie.

Die Figur 6 zeigt die Präparationszeiten (in min) von 2 verschiedenen Säulen-Präparationen. Hierbei zeigt die erfindungsgemäß eingesetzte Vorrichtung (wie im Beispiel 1) mit 20 min eine ebenfalls deutlich kürzere Präparationszeit zur Isolierung von Nukleinsäuren, im Vergleich zu der auf der gleichen Chemie beruhenden Invitek Präparation.

Die Figur 7 zeigt (wie in Beispiel 1) die DNA-Ausbeuten (in µg) der 2 Säulen-Präparationen.

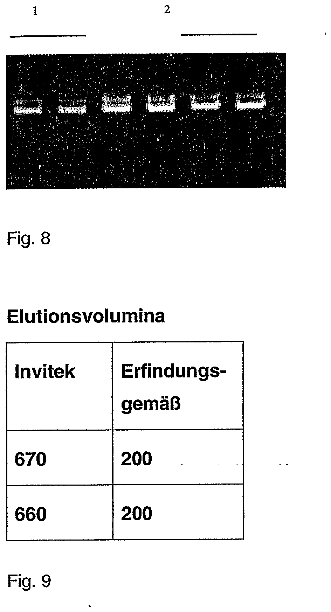

Anhand der in Figur 8 dargestellten Banden im Agarosegel konnte gezeigt werden, dass eine identische Qualität der zwei Referenz-Präparationen erzielt wurde, wobei die Ausbeute von 1282 µg bei erfindungsgemäßer Verwendung der Vorrichtung allerdings deutlich höher ist, als die der Vergleichs-Präparation (Invitek).

Diese Ausbeuten sind mit einer chaotropen Bindechemie nicht zu erreichen, da dort nur Bindestellen auf der inneren Oberfläche der Membran genutzt werden können, was den Einsatz einer Mini-Spin-Säule bzw. des erfindungsgemäßen Verfahrens ausschließt.

In Figur 9 werden die Elutionsvolumina (in µl) von zwei Säulen-Präparationen gegenüber gestellt, woraus eindeutig hervorgeht, dass mit der nach dem erfindungsgemäßen Verfahren eingesetzte Säule im Mini-Format, auch mit einer geringeren Menge Puffer, DNA eluiert werden kann. Durch die enthaltene kleine Membran entsteht so gut wie kein Totvolumen, so dass das erhaltene Elutionsvolumen mit der eingesetzten Puffermenge praktisch identisch ist.

### Beispiel 3:

### Einfluss der GF/D-Membran der erfindungsgemäß eingesetzten Vorrichtung zur Isolierung von Nukleinsäuren während des Wechsels vom Midi-Maßstab auf den Mini-Maßstab; der reduzierten Säulenpräparation.

Es wurde ein synthetisches Lysat aus Resuspendierungs-, Lyse- und Neutralisationspuffer hergestellt. Anschließend wurden je 6 ml dieses "Lysats" mit unterschiedlichen Mengen Plasmid-DNA (10 µg - 250 µg) versetzt, Isopropanol zugegeben und durch die Säule der im erfindungsgemäßen Verfahren eingesetzten Vorrichtung, die je 2 Lagen einer GF/D-Membran (Firma Whatman) enthielt, gesaugt.

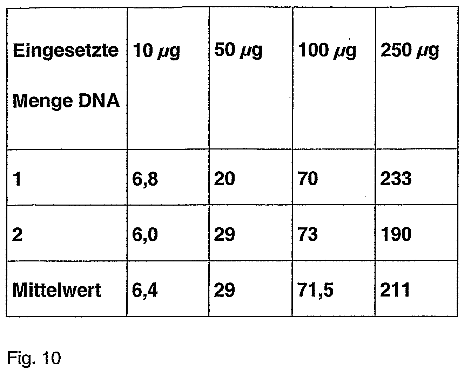

Die Figur 10 zeigt die DNA-Ausbeuten (in µg) der Säulen-Präparation nach Zugabe von ansteigenden Mengen Plasmid-DNA (10 µg - 250 µg). Es konnte gezeigt werden, dass mit steigenden Mengen von Plasmid-DNA (10 µg - 250 µg) die erzielbare DNA-Ausbeute auf 211 µg anstieg.

Die Durchflussgeschwindigkeiten liegen zwischen 10 - 35 ml/min, abhängig vom angelegten Vakuum (ca. 600 - 20 mbar).

Zusätzliche Untersuchungen zeigen, dass sich bei Verwendung von Membranen, die nominelle mittlere Porengrößen von 2 - 5 µm aufweisen, eine hohe Ausbeute mit großer Durchflussgeschwindigkeit erzielen lässt.

### Beispiel 4:

### Durchflussgeschwindigkeiten unter Verwendung von Filtervlies im Maxi-Maßstab

Dieses Experiment zeigt den Einfluss eines Filtervlieses (35 g/m²) auf die Durchflussgeschwindigkeit der erfindungsgemäß eingesetzten Vorrichtung.

Protokoll zur Isolierung von Plasmid-DNA aus *E*. *coli:*
100 ml *E. coli* DH5a - Kultur mit dem Plasmid pCMVβ (DH5α/pCMVβ, Fa. BD Biosciences).

Die *E. coli* Kultur wurde nach den Präparationsschritten gemäß dem in Beispiel 2 beschriebenen Maxi-Protokoll lysiert, das Lysat durch Filtration geklärt und mit Isopropanol versetzt.

Die Lysat/Isopropanol-Mischung wurde anschließend bei 200 mbar durch die Säule gepresst. Die Präparat-Säule wurde mit einem Filtervlies (35 g/m²) bestückt.

Es konnte eine Durchflussgeschwindigkeit von 7,2 ml/min mit der erfindungsgemäß eingesetzten Vorrichtung und dem verwendeten Filtervlies beobachtet werden.

Durch diese verbesserte Verstopfungsresistenz können wesentlich größere Lysatvolumina prozessiert werden, womit das Verfahren auch für größere Maßstäbe benutzt werden kann.

## Patentansprüche

1. Verfahren zur Aufreinigung von Nukleinsäuren, enthaltend DNA, insbesondere Plasmid-DNA, umfassend die folgenden Schritte
a) Einbringen eines Nukleinsäure-Gemisches enthaltend DNA in eine Vorrichtung zur Aufreinigung von Nukleinsäuren umfassend eine Säulenkombination bestehend aus:
i. einem offenen Hohlkörper (1) mit einer Einlassöffnung und einer Auslassöffnung, der auf einem weiteren
ii. offenen Hohlkörper (2) mit einer Einlassöffnung und einer Auslassöffnung, mit einer darin enthaltenen Membran, angeordnet ist, wobei als Membran eine Glasfasermembran mit einer Porengröße in einem Intervall von 2 - 4 µm im Hohlkörper (2) angebracht ist,
b) Binden der DNA-Nukleinsäuren an die Membran in dem Hohlkörper (2) in Gegenwart eines aliphatischen Alkohols mit 1 bis 5 Kohlenstoffatomen,
c) Eluieren der DNA-Nukleinsäuren von der Membran in dem Hohlkörper (2) mit einem an sich bekannten Elutionspuffer.

2. Verfahren nach Anspruch 1, **dadurch gekennzeichnet, dass** als Glasfasermembran eine GF/D Membran verwendet wird.

3. Verfahren nach Anspruch 1 oder 2, **dadurch gekennzeichnet, dass** der Hohlkörper (2) eine zur lonenaustauschchromatographie geeignete Säule ist, wobei die Innenfläche der Säule mit einem zum lonenaustausch befähigten chromatographischen Material beschichtet ist.

4. Verfahren nach einem der Ansprüche 1 bis 3, **dadurch gekennzeichnet, dass** das eingesetzte Volumen an Elutionspuffer mit dem erhaltenen Elutionsvolumen identisch ist.

5. Verfahren nach einem der Ansprüche 1 bis 4, **dadurch gekennzeichnet, dass** das Verfahren ein Verfahren zur Aufreinigung von Plasmid-DNA ist und ein Plasmid-DNA Gemisch in die Vorrichtung eingebracht wird.

6. Verfahren nach einem der Ansprüche 1 bis 5, **dadurch gekennzeichnet, dass** der in Schritt b) eingesetzte aliphatische Alkohol ausgewählt ist aus Ethanol und Isopropanol.

## Claims

1. Method for the purification of nucleic acids, comprising DNA, in particular plasmid DNA, comprising the following steps
a) Introducing a nucleic acid mixture comprising DNA into a device for the purification of nucleic acids comprising a column combination consisting of:
i. an open hollow body (1) with an inlet opening and an outlet opening, which is arranged on a further
ii. open hollow body (2) with an inlet opening and an outlet opening, with a membrane contained therein, wherein as membrane a glass fiber membrane with a pore size in an interval of 2 - 4 µm is fitted in the hollow body (2),
b) Binding the DNA nucleic acids to the membrane in the hollow body (2) in the presence of an aliphatic alcohol with 1 to 5 carbon atoms,
c) Eluting the DNA nucleic acids from the membrane in the hollow body (2) with a known elution buffer.

2. Method according to claim 1, **characterized in that** a GF/D membrane is used as glass fiber membrane.

3. Method according to claim 1 or 2, **characterized in that** the hollow body (2) is a column suitable for ion exchange chromatography, wherein the inner surface of the column is coated with a chromatographic material suitable for ion exchange.

4. Method according to one of claims 1 to 3, **characterized in that** the applied volume of elution buffer is identical with the obtained elution volume.

5. Method according to one of claims 1 to 4, **characterized in that** the method is a method for the purification of plasmid DNA and a plasmid DNA mixture is introduced into the device.

6. Method according to one of claims 1 to 5, **characterized in that** the aliphatic alcohol used in step b) is selected from ethanol and isopropanol.

## Revendications

1. Procédé de purification d'acides nucléiques, contenant de l'ADN, notamment de l'ADN plasmidique, comprenant les étapes suivantes :
a) l'introduction d'un mélange d'acides nucléiques contenant de l'ADN dans un dispositif pour la purification d'acides nucléiques comprenant une combinaison de colonnes constituée par :
i. un corps creux ouvert (1) comprenant une ouverture d'entrée et une ouverture de sortie, qui est agencé sur
ii. un corps creux ouvert supplémentaire (2) comprenant une ouverture d'entrée et une ouverture de sortie, dans lequel est contenue une membrane, une membrane de fibres de verre ayant une taille de pores dans un intervalle allant de 2 à 4 µm étant disposée dans le corps creux (2) en tant que membrane,
b) la liaison des acides nucléiques ADN à la membrane dans le corps creux (2) en présence d'un alcool aliphatique contenant 1 à 5 atomes de carbone,
c) l'élution des acides nucléiques ADN à partir de la membrane dans le corps creux (2) avec un tampon d'élution connu en soi.

2. Procédé selon la revendication 1, **caractérisé en ce qu'**une membrane GF/D est utilisée en tant que membrane de fibres de verre.

3. Procédé selon la revendication 1 ou 2, **caractérisé en ce que** le corps creux (2) est une colonne appropriée pour la chromatographie par échange d'ions, la surface intérieure de la colonne étant revêtue avec un matériau chromatographique apte à l'échange d'ions.

4. Procédé selon l'une quelconque des revendications 1 à 3, **caractérisé en ce que** le volume de tampon d'élution utilisé est identique au volume d'élution obtenu.

5. Procédé selon l'une quelconque des revendications 1 à 4, **caractérisé en ce que** le procédé est un procédé de purification d'ADN plasmidique et un mélange d'ADN plasmidique est introduit dans le dispositif.

6. Procédé selon l'une quelconque des revendications 1 à 5, **caractérisé en ce que** l'alcool aliphatique utilisé à l'étape b) est choisi parmi l'éthanol et l'isopropanol.
